(19) Europäisches Patentamt
European Patent Office
Office européen des brevets

(11) Publication number: **0 098 557**
**B1**

(12) **EUROPEAN PATENT SPECIFICATION**

(45) Date of publication of patent specification: **10.06.87**

(51) Int. Cl.$^4$: **G 01 N 33/569**

(21) Application number: **83106453.0**

(22) Date of filing: **01.07.83**

(54) **Preparation for the diagnosis of chlamydial infections and for the production of chlamydial group-specific antibodies.**

(30) Priority: **02.07.82 FI 822348**

(43) Date of publication of application:
**18.01.84 Bulletin 84/03**

(45) Publication of the grant of the patent:
**10.06.87 Bulletin 87/24**

(84) Designated Contracting States:
**AT BE CH DE FR GB IT LI NL SE**

(56) References cited:
**US-A-4 118 469**

**SCIENCE, vol. 220, no. 4603, 17th June 1983, pages 1279-1281 M. NURMINEN et al.: "The genus-specific antigen of Chlamydia: Resemblance to the lipopolysaccharide of enteric bacteria"**

**CURRENT TOPICS IN MEMBRANES AND TRANSPORT, vol. 17, 1982, pages 79-151 O. LÜDERITZ et al.: "Lipopolysaccharides of gram-negative bacteria"**

(73) Proprietor: **Orion Corporation, Ltd.**
**Nilsiänkatu 10-14**
**SF-00510 Helsinki 51 (FI)**

(72) Inventor: **Mäkelä, Pirjo Helena**
**Pihlajatie 50-52 B 40**
**SF-00510 Helsinki 51 (FI)**
Inventor: **Leinonen, Maija Kaarina**
**Kylätie 20 A 2**
**SF-00230 Helsinki 32 (FI)**
Inventor: **Nurminen-Kalliokoski, Marjatta Hellevi**
**Virolahdentie 5**
**SF-00950 Helsinki 95 (FI)**
Inventor: **Saikku, Pekka Auvo Ilmari**
**Steniuksentie 12 A**
**SF-00320 Helsinki 32 (FI)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 098 557 B1**

**Description**

The present invention relates to a preparation for the diagnosis of chlamydial infections. This preparation contains at least one lipopolysaccharide (LPS) isolated from rough (R) mutants of the chemotype Re (as described below) of gram-negative bacteria. Antibodies to this LPS cross-react with the group antigen originating from chlamydia species.

R mutants are a well-known class of bacterial mutants (reviews for example Lüderitz et al., pp. 105—228 in "Comprehensive Biochemistry", M. Florkin and E. H. Stotz, Eds., Vol. 26A Elsevier, Amsterdam 1968; Mäkelä and Stocker, Ann. Rev. Genetics 1969, 3, 291—322; Mäkelä and Stocker in "Genetics as a Tool in Microbiology", Glower and Hopwood, Eds., Soc. Gen. Microbiol. Symp. 31, Cambridge University Press 1981) in which a step in the biosynthesis of LPS is defective and blocks the completion of the molecule. The R mutants can be classified into chemotypes according to their LPS structure (Fig. 4 and pages 156—171 in Lüderitz et al. 1968 cited above, and Fig. 4 in Lüderitz et al, "Current Topics in Membrane and Transport", 17, 79—151, 1982). In this classification a chemotype Re or "Re mutant" is defined as a mutant whose LPS consists of lipid A and KDO (3-deoxy-D-manno-octulosonic acid, previously named 2-keto-3-deoxy-octonate) with no other monosaccharide substituents. For this reason Re mutants are often referred to as "heptoseless" since they are devoid of even heptose, the monosaccharide that in most LPS is the next unit linked to KDO. Such Re mutants were first described in the genus *Salmonella* (both *S. typhimurium* and *S. minnesota*) but have been later found in other species (*Escherichia coli, Proteus mirabilis*), and can probably be isolated from many others because the LPS structure in the immediate vicinity of lipid A is very conservative throughout the gram-negative bacteria (Lüderitz et al. 1982 cited above).

*Chlamydia* are very small, obligate intracellular gram-negative bacteria. Two species are known, i.e. *Chlamydia trachomatis* and *Chlamydia psittaci.* These species are common parasites in mammals and birds. They cause various diseases in humans and animals. Chlamydia are, for example, responsible for nonspecific urethritis (NSU), ovarian infections, ocular infections, pneumonia and reactive arthritis. In animals, chlamydia cause abortion and epidemics of diarrhoea. Antichlamydial dry therapy, exists but because of cumbersome diagnostic methods the therapy of chlamydial infections is difficult.

Chlamydial diagnosis is based on bacterial culture and/or serological identification of antibodies. Chlamydia cannot be identified by conventional bacteriological culture methods on ordinary media. The cultivation of chlamydia must be carried out in living cells, e.g. in incubated eggs or tissue cultures. This work has to be carried out in specialized virological laboratories and may inolve a considerable risk of infection.

The best known serological methods used for the diagnosis of chlamydial infections are the complement fixation test, using a group antigen, countercurrent immunoelectrophoresis methods, and various immunofluorescence methods. Serological diagnostic methods for chlamydia are described in the patent literature, e.g. in US Patent 4 118 469, European Patent 17460 and British patent 2 047 889. The isolation of chlamydial antigens and their use as vaccines are described in US Patents 4 118 469 and 4 039 657, 4 096 035, 4 271 146 and 4 267 170.

All the diagnostic methods reported in the patent publications listed above share a common disadvantage, i.e. the production of the antigen. Chlamydial antigens have to be produced by cultivating chlamydia in tissue cultures or in eggs. These methods are characterized by a low yield and inevitable contamination by substances from the associated animal cells, which interfere with the sensitive immunological tests. Because of the impurities in such antigen preparations, a control antigen is needed to allow assessment of nonspecific reactions. The preparation of chlamydial antigen preparations in living cells is difficult and slow and, in addition to being risky to health, is expensive.

It is an object of this invention to provide a preparation for the diagnosis of chlamydial infections and for the production of chlamydial group-specific antibodies which eliminates all the drawbacks and problems described above associated with the production of chlamydial antigen preparations. Specifically, the invention is based on the finding that lipopolysaccharides (LPS) derived from R mutants of the chemotype Re of gram-negative bacteria react in both directions with the chlamydia glycolipid (also called group antigen). This cross-reaction means that the Re-type lipopolysaccharides react with antibodies against chlamydial glycolipids and *vice versa* the glycolipid antigen of chlamydia species react with antibodies to the Re-lipopolysaccharides. The Re mutants of the gram-negative bacteria mentioned above are easily cultivated using known bacteriological methods. Both solid and liquid culture media can be used. Production of bacterial mass on a large scale, e.g. in fermentors, can be carried out. The Re lipopolysaccharides concerned can subsequently be isolated from these Re mutants of gram-negative bacteria using known methods (see Galanos et al., Eur. J. Biochem., 9, 245—249, 1969).

The preparation of the present invention containing a lipopolysaccharide (LPS of chemotype Re as defined by Lüderitz et al., 1968 and 1982, as cited above) can be used for the production of group-specific antibodies against chlamydia, for diagnostic purposes, for example, or for the identification of antibodies to chlamydia group antigen in specimens such as serum, faeces, or nasal, laryngeal or urethral mucus. The preparation of the invention can be used as antigen in serological or immunological methods used in the diagnosis of chlamydial infections, such as the complement fixation test, countercurrent immunoelectrophoresis, passive haemagglutination, hemolysis-in-gel (HIG), enzyme immunoassay (EIA), immunofluorescence (FIA) and radioimmunoassay (RIA). The use of the preparation of the present invention is not,

however, limited to the methods described above but can, in principle, be used in all serological or immunological methods in which the antibodies to the group specific antigen of chlamydia need to be identified. Antiserum to the group specific chlamydial antigen can be produced directly by immunization of animals with the Re-mutants of a gram-negative bacterium without isolation of their lipopolysaccharide.

The Re lipopolysaccharide preparations of the present invention are easy and inexpensive to produce. They are suitable for mass production, and can be used in diagnosis and in immunological or serological methods in many different ways. In addition, they are free of cellular protein and other non-LPS constituents. Control antigens are, therefore, not needed in order to determine interference of extraneous material from a tissue culture or an egg preparation, in contrast to the situation when group antigens of chlamydial origin are used.

It is a characteristic of the preparation of the present invention that the LPS is derived from the basic component of the lipopolysaccharides of those gram-negative bacteria in which the core component of the polysaccharide is connected via KDO to the lipid A, as shown in the general formula I

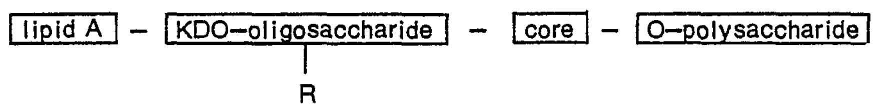

(I)

according to Lüderitz et al., 1982 as cited above. The KDO-oligosaccharide contains two or three KDO-groups and at least one of these KDO-groups is substituted by R.

The structures of the lipid A component, core component, and O-polysaccharide component can vary in different bacterial strains. The structure of the KDO-oligosaccharide containing two or three KDO-groups is fairly constant. The lipid A component of the lipopolysaccharide illustrated in formula I contains a glucosamine disaccharide, variously substituted at the hydroxyl groups by fatty acid groups, 4-aminoarabinose phosphate groups and diphosphoryl ethanolamine groups. At the amino groups it is substituted by fatty acid groups. KDO means 3-deoxy-D-manno-octulosonic acid, the structure of which is shown in formula II

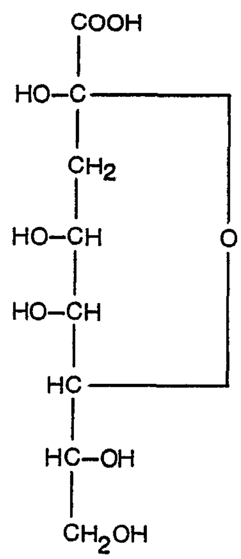

(II)

The two or three KDO molecules present in the lipopolysaccharide shown in formula I are joined to each other and to the lipid A moiety by ketosidic linkage. R denotes a hydrogen atom or a phosphate group or a diphosphoryl lower alkanolamine group, preferably, a diphosphorylethanolamine group. The core component of the lipopolysaccharide comprises a polysaccharide, the composition of which varies in different bacterial groups. In wild type *Salmonella* species, for example, the core polysaccaride contains at least glucosamine, glucose, galactose, and heptose. The o-polysaccharide component of the lipopolysaccharide comprises n monosaccharides, where n is an integer having the value 0 or at least 1. The structure of the monosaccharides may vary freely. R mutants have variously defective LPS. Among these R mutants the chemotype Re mutants synthesize a Re lipopolysaccharide the structure of which can be represented by the general formula III as

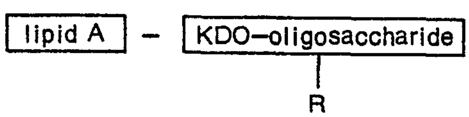

(III)

wherein the KDO-oligosaccharide contains two or three KDO-groups and at least one of these KDO-groups is substituted by R.

On the basis of chemical analysis, the molecular weight of a Re lipopolysaccharide is about 3000.

The Re-LPS migrates in electrophoresis in 15% sodium dodecylsulfate-polyacrylamide gel (the SDS-PAGE method as described by Laemmli, Nature 227, 680—685, 1970), as a characteristic band. The speed of migration (as indicated by the final position of the band after electrophoresis) is similar to that of the glycolipid group antigen of *Chlamydia trachomatis* and clearly different from the speed of migration of LPS of chemotype $Rb_2$ which has a molecular mass of approximately 4000 d and has 6 monosaccharides of the core in addition to the lipid A-KDO-oligosaccharide (see Lüderitz et al., 1982, as cited above for the $Rb_2$ structure). In the gel the LPS bands can be visualized after staining with silver nitrate (Tsai and Frasch, Anal. Biochem. 119, 115—119, 1982) or with immunoblotting (Towbin et al., Proc. National. Acad. Sciences, 76, 4350—4354, 1979). Such a gel (after silver nitrate staining) is shown in Fig. 1 in which lane 1 is $Rb_2$-LPS from *S. typhimurium* $Rb_2$ mutant strain SH5014 (Nurminen et al., J. Bact., 127, 941—955, 1976), lane 2 is Re-LPS from *S. typhimurium* Re mutant strain SL1102, and lane 3 is glycolipid from *Chlamydia trachomatis.* The samples were applied on top of the gel; only the lower part of the gel with the small-molecular weight LPS bands is shown.

Some of the fatty acid groups and 4-aminoarabinose phosphate groups may be removed by treatment with a weak alkali (preferably 0.16 N NaOH, 1h, 56°C), but the KDO component remains. The Re lipopolysaccharide shown in formula III when treated with weak alkali retains its immunogenic properties, and can be used with advantage to identify antibodies to chlamydial group antigen by various immunological or serological methods. Boiling in weak acid (preferably 0.1 N HC1) removes the KDO part, with the result that the immunodeterminants important for chlamydial diagnosis are destroyed.

The antisera (antibodies) produced against Re lipopolysaccharides or against whole Re mutant bacteria in experimental animals also react with glycolipid antigens isolated from *Chlamydia* species.

For producing the preparations of the present invention Re mutants are used which synthesize heptose-less lipopolysaccharides of the kind shown in formula III. The Re mutants are produced according to known methods from gram-negative parent bacteria which synthesize lipopolysaccharides of the type shown in formula I. Such Re mutants can be derived from various gram-negative bacteria, e.g. *Salmonella typhimurium* (Re mutant SL1102) (Wilkinson et al, J. Gen. Microbiol. 70, 527—554, 1972), *Salmonella minnesota* (Re mutant R595) (Lüderitz et al., Ann. N.Y. Acad. Sci. 133, 349—374, 1966) *Escherichia coli* K-12 (Re mutant D21f2) (Mayer et al., Eur. J. Biochem. 66, 357—368, 1976) and *Proteus mirabilis* (Re mutant R45) (Kotelko et al., J. Hyg. Epidemiol. Microbiol. Immunol. 21, 271—284, 1977).

It should be noted that the lipopolysaccharide preparations of the present invention can also be produced from gram-negative bacteria other than those mentioned above. The main requirement is that the gram-negative bacteria and the Re mutants produced from them comply with the conditions mentioned above conerning the general formula of the lipopolysaccharides.

The bacteria mentioned above do not require specific nutrients in the culture media. In addition, all gram-negative bacteria in which Re mutation has taken place are very sensitive to detergents, bile acids and several hydrophobic antibiotics to which the parent strains are resistant. Re mutants are, therefore, viable under laboratory conditions only.

Since the bacteria do not require specific nutrients they can be grown on a wide variety of media. One convenient medium is an enriched Salmonella-agar (Schlecht and Westphal, Zentralblatt für Bakteriologie, Parasitenkunde, Infektionskrankheiten und Hygiene, 200 (1966), 241—259). The Re mutants are grown overnight on the culture medium and the bacteria are collected from the surface of the agar plate by rinsing with water. The cells are centrifuged (5000 rpm, 10 min) and washed with distilled water. The use of distilled water at this stage is essential for the successful isolation of the lipopolysaccharide. Isolation of the lipopolysaccharide from the Re mutant is carried out according to the method described by Galanos et al., Eur. J. Biochem. 9, 245—249, 1969 but other known methods may also be used; see Westphal and Jann, in Methods in Carbohydrate Chemistry, Editors R. L. Whistler, J. N. BeMiller and M. L. Wolfram, Academic Press, New York, Vol. 5, p. 83. Alkali treatment of the LPS can be performed as described in Handbook of Micromethods for the Biological Sciences, Keleti and Lederer, Eds, 1974, p. 133—135.

The propagation of Re mutants, the isolation and purification of the Re lipopolysaccharide preparations and their alkali treatment are described in greater detail in the following examples.

## Example 1

Re mutant R595 of *Salmonella minnesota* (see Lüderitz et al., 1966, cited above).

### A. Culture of bacteria

The bacteria are grown overnight at 37°C on agar plates prepared from 10 litres of enriched Salmonella-agar (see Schlecht and Westphal, 1966, cited above).

The bacteria are collected from the surface of the agar plate by rinsing with distilled water (500 ml), and transferred into a centrifuge bottle, centrifuged (5000 rpm, 10 min), washed once with water, and freeze-dried. The yield is about 30 g.

B. Isolation of the Re lipopolysaccharide (Re-LPS)

a) the lipopolysaccharide is extracted at icebath temperature from the freeze-dried bacterial mass using a mixture of phenol: chloroform: petrolether (2:5:8);

b) from the extract the chloroform and petrolether are distilled off and the lipopolysaccharide is precipitated by addition of water;

c) the lipopolysaccharide is purified by dissolving the precipitate in water at 45°C, and pelleting it by centrifuging at 100000 × g;

d) the lipopolysaccharide is stored at room temperature after freeze-drying;

e) the yield of lipopolysaccharide is about 5% of the bacterial dry weight.

C. Alkali treatment of the Re-LPS

a) 10 mg of the LPS is solubilized in 1 ml of 0.25 N sodium hydroxide;

b) the solution is incubated at 56°C for 60 min;

c) the solution is cooled to room temperature;

d) the solution is centrifuged (2000 rpm, 15 min) at 4°C;

e) the supernatant is collected and neutralized with 1 N acetic acid;

f) the solution is dialyzed overnight against water;

g) the preparation is freeze-dried.

D. Immunization with the isolated lipopolysaccharide

Pure lipopolysaccharides as such are not good immunogens but nned to be complexed with a carrier protein.

For example 1 mg of Salmonella-porin-protein (Kuusi et al., Inf. Imm., 34 (1981), 328—329; the porin-protein complex is an outer membrane protein) and 0.1 mg of Re lipopolysaccharide are mixed in 5 ml of 0.25% sodium lauryl sulphate (SDS). The mixture is dialysed until SDS is removed.

Antibodies are produced by known immunization methods, e.g. by injecting 0.5 ml of the mixture 4 times at 2 week intervals into the foot pads of a rabbit. Serum is collected 10 days after the last injection.

E. Immunization with whole bacteria.

The bacteria are grown in nutrient broth up to the logarithmic growth phase, collected by centrifugation and washed once with physiological saline. They are suspended in physiological saline ($10^{10}$ bacteria/ml), heated for 1 h at 100°C; then 0.5% formalin is added. Rabbits are immunized by injecting 0.5 ml of this preparation in the foot pads 7 times at one week intervals. The serum is collected 10 days after the last injection.

Using the method described in A it is also possible to cultivate other Re mutants prepared from other gram-negative bacteria. Antisera against these bacteria can be produced using the method described in E. The lipopolysaccharides can be isolated from the bacteria according to the method described in B. Antisera against the lipopolysaccharides can be produced by the method described in D.

In the following paragraphs, details of tests and results of these tests are presented which demonstrate the usefulness of the lipopolysaccharide preparations for the diagnosis of chlamydial infections.

Test 1

Reaction of antibodies produced against Re mutants of gram-negative bacteria with chlamydial glycolipids.

The cross-reactions of antibodies against Re mutants of gram-negative bacteria were investigated using the enzyme immunoassay (EIA) (see Koskela and Leinonen, Ped. Inf. Dis., *1*, 245—252, 1982). The glycolipid isolated from chlamydia was used as an antigen. The results (see Table 1) show that antisera prepared by the immunization of rabbits with *Salmonella* Re mutant strains (*S. typhimurium, S. minnesota*) and Re-LPS-porin complex contain high-titer antibodies against chlamydial glycolipids. The antibody titers against chlamydial glycolipid in *S. typhimurium* and *S. minnesota* Re mutant antisera as determined by the EIA method are shown in Table 1.

TABLE 1

| Immunogen | EIA titer |
|---|---|
| *S. typhimurium* Re | 8500 |
| *S. typhimurium* Re | 1000 |
| *S. minnesota* Re | 2000 |
| *S. minnesota* Re-LPS-porin complex | 1750 |
| Normal serum | <100 |
| *S. typhimurium* Rd2* | <100 |

* one residue of LPS core components is present in the mutant.

Test 2

Reaction of anti-chlamydia antibodies with lipopolysaccharide preparations (Re-LPS and alkali-treated Re-LPS) isolated from Re mutants of gram-negative bacteria A. EIA and other solid phase methods.

Microtiter plates (EIA plates, A/S Nunc., Roskilde, Denmark) were coated using the following Re-LPS concentrations: 1, 5, 10 and 20 μg/ml of phosphate-buffered saline solution (PBS, pH 7.4) by incubation at 37°C overnight. EIA determinations were carried out and the results (EIA titers) calculated as previously described in detail (see Koskela and Leinonen, J. Clin. Path., 34, 93—98, 1981, Koskela et al., Ped. Inf. Dis., 1, 245—252, 1982). The alkaline phosphatase labelled anti-immunoglobulin conjugates were commercial preparations (antihuman IgG, IgM and IgA, anti-rabbit IgG, anti-mouse IgG, Orion Diagnostica, Helsinki, Finland).

The optimal Re-LPS and alkali-treated Re-LPS concentrations giving the highest EIA titers in antiserum to Re mutant, and giving a negative result in non-immunized rabbit serum was found to be 10 μg/ml. This Re-LPS concentration was used for coating the plates in later studies.

Using the EIA method three antisera produced by immunizing rabbits with chlamydia were tested against Re-LPS isolated as described in Example 1 from Re mutants of *S. typhimurium* SL1102, *S. minnesota* R595, *E. coli* D21f2 and *P. mirabilis* R45 cited above. The EIA-antibody titers against these Re-LPS preparations in the three rabbit antisera against chlamydia and in rabbit antiserum against *S. minnesota* Re mutant (Example 1 E) and in normal rabbit serum are shown in Table 2.

TABLE 2

| Antiserum | EIA titer to LPS isolated from Re mutant of *S. typhim.* | *S. minnes.* | *E. coli* | *Proteus* |
|---|---|---|---|---|
| *Chlamydia* 1 | 2150 | 2111 | 2180 | 2100 |
| *Chlamydia* 2 | 2750 | 2600 | 1920 | 2760 |
| *Chlamydia* 3 | 1400 | 1830 | 1250 | 230 |
| *S. minnesota* Re mutant | 490 | 140 | 940 | 1370 |
| NRS* | <100 | <100 | <100 | <100 |

* NRS = normal rabbit serum

Monoclonal mouse antibodies (produced by the hybridoma technique, Köhler and Milstein, Nature, 256, 495—497, 1975) against chlamydia which react in the EIA test with chlamydial glycolipid preparations also react with the Re-LPS preparations. The EIA reactions of monoclonal antibodies to chlamydial glycolipid, Re-LPS and alkali-treated Re-LPS are shown in Table 3.

TABLE 3

| Monoclonal antibody | EIA reaction, when the antigen is Chlamydial glycolipid | *Salmonella* Re-LPS | Alkali-treated Re-LPS |
|---|---|---|---|
| 1 | + | + | + |
| 2 | + | + | + |
| 3 | + | + | + |
| 4 | + | + | + |

Because monoclonal antibodies are specific to one antigenic determinant, the reactions of monoclonal antibodies specific to chlamydial glycolipids with Re-LPS indicate a common antigenic structure in the chlamydial glycolipid and Re-LPS.

Radio- and fluoroimmunoassays (RIA and FIA) can be used in these tests instead of EIA, because they differ from the EIA only by the label of the second antibody (radioisotope in RIA, fluorochrome in FIA and enzyme in EIA).

B. Countercurrent immunoelectrophoresis (CIE)

The antiserum from a rabbit immunized with *Chlamydia (Chlamydia* 1, Table 2) was tested against Re-LPS (from *S. typhimurium* SL1102) and alkali-treated Re-LPS by using the CIE-method (Leinonen, J. Clin. Microbiol., 11, 135—140, 1980). The results are shown in Fig. 2. Re-LPS (A) and alkali-treated Re-LPS (B) in three different concentrations 100 µg/ml, 10 µg/ml, and 1 µg/ml (1, 2 and 3) form a clear-cut precipitation line with chlamydial antiserum (C).

C. Double immunodiffusion

The antiserum from a rabbit immunized with *Chlamydia (Chlamydia* 1, Table 2) was tested against Re-LPS (from *S. typhimurium* SL1102) and alkali-treated Re-LPS by using double immunodiffusion (Fig. 3). The wells were punched out from the 0.9% agarose gel, containing 0.15% deoxycholate (DOC). The wells contained 100 µg/ml 0.15% DOC of chlamydial glycolipid (1,4), Re-LPS (2) and alkali-treated Re-LPS (3). The center well contained chlamydial antiserum. The precipitation lines formed between this serum and Re-LPS, alkali-treated Re-LPS and chlamydial glycolipid fuse, showing the reaction of identity (according to Ouchterlony and Nilsson, 1978, Handbook auf Experimental Immunology, Ed. D. M. Weir, 3e Chapter 19, Blackwell Scientific Publications, Oxford).

D. Passive hemagglutination

Sheep red blood cells were sensitized with Re-LPS (from *S. typhimurium* SL1102) or with the alkali-treated Re-LPS as described (Handbook of Micromethods for the Biological Sciences, Keleti and Lederer, Eds., pp. 134—135, 1974). The washed cells were suspended in phosphate buffered saline to form a 0.5% suspension. 25 µl of this suspension are mixed with 75 µl of serial dilutions of the antisera to be tested in U-shaped wells of a microtiter plate. The agglutination was read by naked eye after incubation for 30 min at 37°C followed by overnight incubation at room temperature. Positive agglutinations were not observed with a normal rabbit serum, whereas the rabbit anti-chlamydial serum (*Chlamydia* 1, as in Table 2) agglutinated these cells in a high titer (Table 4). Untreated sheep red blood cells were not agglutinated.

TABLE 4

Passive hemagglutination titers with sheep red blood cells sensitized with Re-LPS

| | Sensitized with | | Unsensitized |
|---|---|---|---|
| Serum | Re-LPS | alkali-treated Re-LPS | |
| *Chlamydia* 1 | 1024 | 256 | <2 |
| Normal rabbit | <2 | <2 | <2 |

E. Hemolysis in gel

Chicken red blood cells were sensitized with the alkali-treated Re lipopolysaccharide of *S. typhimurium* SL1102 as described in D.

The Re-LPS-treated red blood cells were mixed with melted agarose and complement, poured into plastic dishes and allowed to solidify as a gel plate as described by Väänänen and Vaheri, J. Med. Virology, 3, 245—252, 1979 and Väänänen J. Virol. Methods 4, 117—126, 1982. Wells of a diameter of 2 mm were then punched in the gel. 6 µl of heat inactivated (56°C, 30 min) human serum were applied to the wells. The plate was incubated overnight at 37°C and the diameters of the hemolytic zones were measured. The results were compared with the results obtained by the anti-chlamydial indirect fluorescence antibody test (IFAT, Weller and Coons, Proc. Soc. Exp. Biol. Med., 86, 789, 1954 and Gardner and McQuillan (Eds.), Rapid Virus Diagnosis. Application of Immunofluorescence, Butterworths, London, p. 60), the routine method of measuring chlamydial antibodies in human sera. The results obtained by these methods (HIG and IFAT) are in good agreement as shown in Table 5.

TABLE 5

HIG assay for anti-Re in human sera compared with anti-chlamydia IFAT (the chicken red blood cells were sensitized with alkali-treated Re-LPS).

| | Methods used | |
|---|---|---|
| Human serum | HIG diameter (mm) | IFAT (titer) |
| normal | 0 | <16* |
| patient | 6 | 256 |

* < 16 means a negative result

F. Latex-Agglutination

Covalent fixation of the Re lipopolysaccharide (from *S. typhimurium* SL1102) and alkali-treated Re lipopolysaccharide to latex particles was effected with a water-soluble carbodiimide (CDI) as described by Goodfriend et al., Science, 144, 1344—1346, 1964.

a) 0.5 ml of carboxylated polystyrene latex particles (0.8 μm diameter, 100 g/l, Estapor®, Rhône-Poulenc, France) were washed two times in 5 ml of isotonic saline buffered by borate (20 mmol/l, BBS, pH 8.1), centrifuged and resuspended in 1 ml of this buffer;

b) the latex suspension was activated by being stirred for 45 min at room temperature with 25 mg of 1-ethyl-3-(dimethylaminopropyl)carbodiimide hydrochloride (Sigma Chemical Co.);

c) after centrifugation and resuspension in 1 ml of BBS the activated latex was incubated overnight at room temperature and under gentle agitation with different amounts (150 μg, 250 μg, 300 μg and 500 μg) of Re-LPS and alkali-treated Re-LPS;

d) after incubation 250 μl of 1% bovine serum albumin in glycine buffered saline (GBS-BSA, pH 8.2) were added and the latex was centrifuged, washed three times with GBS—BSA, resuspended in 5 ml of GBS-BSA and sonicated for a few seconds;

e) the latex reagents were stored at 4°C.

The rabbit anti-chlamydia serum (*Chlamydia* 1, Table 2) was tested for its ability to agglutinate the latex particles. A normal rabbit serum (NRS) served as a control. The latex tests were performed by mixing 25 μl of the antiserum or its dilutions and 25 μl of the latex reagent on a glass slide. After tilting for 2 min the slides were examined against a dark background for agglutination. The results are shown in Table 6.

TABLE 6
Latex agglutination titers of rabbit sera with particles sensitized with Re-LPS

| Latex reagent coated with | Serum: *Chlamydia* 1 | Serum: NRS |
|---|---|---|
| Re-LPS | 80 | 0 |
| alkali-treated Re-LPS | 80 | 0 |
| neither | 0 | 0 |

Test 3

Use of Re-LPS preparations in the diagnosis of chlamydial infections.

A. Non-gonococcal urethritis (NGU)

The antibody titers were determined in paired acute and convalescent phase sera obtained from 17 patients with NGU using the EIA method with Re-LPS from *S. typhimurium* SL 1102 as antigen (test 2 A). *Chlamydia* isolation was positive in all cases. The results of antibody determinations in NGU patients using the EIA method are shown in Table 7. The antibody response was considered positive if the antibody titer changed (<2-fold increase or decrease) between the paired sera, or if IgM and/or IgA antibodies were found in the acute phase serum.

TABLE 7
EIA antibody titers with Re-LPS in paired sera of patients with non-gonococcal urethritis (*Chlamydia* isolation positive)

| Criteria of positive response | No. of patients | No. of positive findings: IgG | IgA | IgM | Total |
|---|---|---|---|---|---|
| Titer change (paired sera) | 17 | 13 | 13 | 13 | 15 |
| IgM or IgA demonstrable in acute serum | 17 | | 15 | 14 | 17 |

Serological diagnosis was achieved in all cases in which chlamydia had been isolated, either by demonstration of a change in antibody titer and/or demonstration of acute phase antibodies of immunoglobulin class IgM and/or IgA.

B. Pelvic inflammatory disease (PID)

Acute and convalescent sera from 8 PID patients and acute sera from 5 PID patients with positive chlamydia culture were tested using the EIA method, with Re-LPS from *S. typhimurium* SL1102 as the antigen. In all cases, a serological diagnosis was possible either on the basis of antibody titer change or demonstration of acute phase antibodies of the immunoglobulin classes IgM and/or IgA.

**Claims for the Contracting States: BE CH DE FR GB IT LI NL SE**

1. A preparation for the diagnosis of chlamydial infections and for the production of chlamydial group-specific antibodies, containing a lipopolysaccharide of chemotype Re isolated from Re mutants of gram-negative bacteria, cross-reacting (reacting in both directions) with chlamydial glycolipid.

2. The preparation according to claim 1, characterized in that the preparation has been isolated from Re mutants of gram-negative bacteria which synthetize an Re-lipopolysaccharide as illustrated in formula I

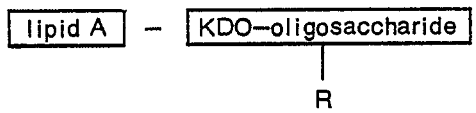

(I)

wherein the KDO-oligosaccharide contains two or three KDO-groups and at least one of these KDO-groups is substituted by R, the lipid A component contains a glucosamine disaccharide which is variously substituted at the hydroxyl groups by fatty acid groups, 4-aminoarabinose phosphate groups and diphosphorylethanolamine groups, and at the amino groups it is substituted by fatty acid groups, KDO signifies 3-deoxy-D-mannose-octulosonic acid of the formula II,

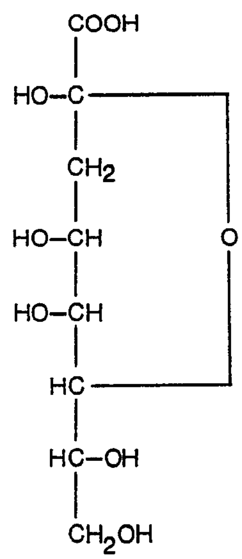

(II)

and R denotes a hydrogen atom or a phosphate group or a diphosphoryl lower alkanolamine group, and where the Re-LPS shown in formula I cross-reacts with chlamydial glycolipids.

3. A preparation according to claim 1 or 2, characterized by the fact that the product shown in formula I has been treated with alkali.

4. A preparation for the production of chlamydial group specific antibodies, characterised in that it comprises Re mutants of gram-negative bacteria whose LPS has not been isolated.

5. The use of the preparation according to any one of claims 1 to 3 for the production of chlamydial group-specific antibodies.

6. The use of the preparation according to any one of claims 1 to 3 for the detection of chlamydial group-specific antibodies by serological or immunological methods.

**Claims for the Contracting State: AT**

1. A method for the production of a preparation for the diagnosis of chlamydial infections and for the production of chlamydial group-specific antibodies, characterized in that a lipopolysaccharide of chemotype Re cross-reacting (reacting in both directions) with chlamydial glycolipid is isolated from Re mutants of gram-negative bacteria.

2. The method according to claim 1, characterized in that the isolation is effected from Re mutants of gram-negative bacteria which synthetize an Re-lipopolysaccharide as illustrated in formula I

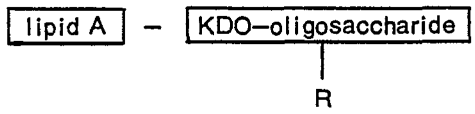

(I)

wherein the KDO-oligosaccharide contains two or three KDO-groups and at least one of these KDO-groups is substituted by R, the lipid A component contains a glucosamine disaccharide which is variously substituted at the hydroxyl groups by fatty acid groups, 4-aminoarabinose phosphate groups and diphosphorylethanolamine groups, and at the amino groups it is substituted by fatty acid groups, KDO signifies 3-deoxy-D-mannose-octulosonic acid of the formula II,

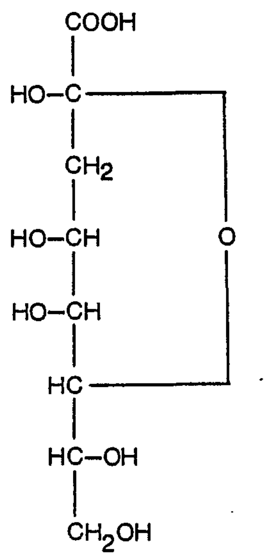

(II)

and R denotes a hydrogen atom or a phosphate group or a diphosphoryl lower alkanolamine group, and where the Re-LPS shown in formula I cross-reacts with chlamydial glycolipids.

3. The method according to claim 1 or 2, characterized in that the product shown in formula I is treated with alkali.

4. A method for the production of chlamydial group specific antibodies characterized in that the Re mutant of gram-negative bacteria, whose LPS has not been isolated, are grown to the logarithmic phase, collected by centrifugation washed with physiological saline, suspended in physiological saline, heat- and formalin-inactivated, injected into rabbits, and the antibodies are collected from the rabbit serum.

5. The method according to claim 4, characterized in that isolated LPS complexed with a carrier protein is used for immunization.

6. The use of the preparation according to any of claims 1 to 3 for the detection of chlamydial group-specific antibodies by serological or immunological methods.

**Patentansprüche für die Vertragsstaaten: BE CH DE FR GB IT LI NL SE**

1. Präparat zur Diagnose von Chlamydia-Infektionen und zur Herstellung gruppenspezifischer Chlamydia-Antikörper, das ein Lipopolysaccharid des Chemotyps Re enthält, das aus Re-Mutanten gram-negativer Bakterien isoliert wurde und mit dem Chlamydia-Glykolipid kreuzreagiert (in beiden Richtungen reagiert).

2. Präparat nach Anspruch 1, dadurch gekennzeichnet, daß es aus Re-Mutanten gram-negativer Bakterien isoliert wurde, die ein Re-Lipopolysaccharid der Formel I

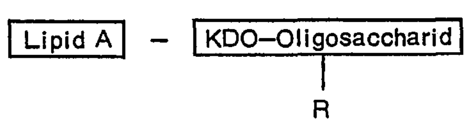

(I)

synthetisieren, in der das KDO-Oligosaccharid zwei oder drei KDO-Gruppen enthält und mindestens eine dieser KDO-Gruppen mit dem Rest R substituiert ist, der Lipid-A-Bestandteil ein Glukosamin-Disaccharid

enthält, das an den Hydroxylgruppen verschiedenartig substituiert ist durch Fettsäurereste, 4-Aminoarabinosephosphatreste und durch Diphosphoryläthanolaminreste, und das an den Aminogruppen durch Fettsäurereste substituiert ist, KDO eine 3-Desoxy-D-mannoseoctulosonsäure der Formel II

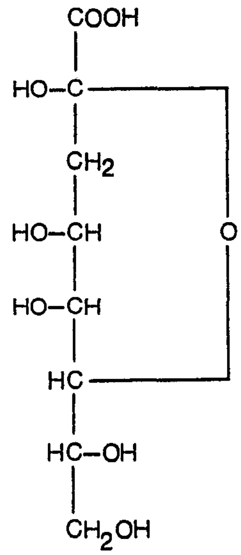

(II)

bedeutet, und in der R ein Wasserstoffatom, eine Phosphatgruppe oder eine Diphosphorylniederalkanolamingruppe ist, und das Re-LPS der Formel I mit Chlamydia-Glykolipiden kreuzreagiert.

3. Präparat nach Anspruch 1 oder 2, dadurch gekennzeichnet, daß die Verbindung der Formel I mit Alkali behandelt wurde.

4. Präparat zur Herstellung von gruppenspezifischen Chlamydia-Antikörpern, dadurch gekennzeichnet, daß es Re-Mutanten gram-negativer Bakterien enthält, deren LPS nicht isoliert worden war.

5. Verwendung des Präparats nach einem der Ansprüche 1 bis 3 zur Herstellung von gruppenspezifischen Chlamydia-Antikörpern.

6. Verwendung des Präparats nach einem der Ansprüche 1 bis 3 zur Nachweis gruppenspezifischer Chlamydia-Antikörper durch serologische oder immunologische Verfahren.

**Patentansprüche für den Vertragsstaat: AT**

1. Verfahren zur Herstellung eins Präparats zur Diagnose von Chlamydia-Infektionen und zur Herstellung von gruppenspezifischen Chlamydia-Antikörpern, dadurch gekennzeichnet, daß man ein Lipopolysaccharid des Chemotyps Re, das mit dem Chlamydia-Glykolipid kreuzreagiert (in beiden Richtungen reagiert) aus Re-Mutanten gram-negativer Bakterien isoliert.

2. Verfahren nach Anspruch 1, dadurch gekennzeichnet, daß man die Isolierung ausgehend von Re-Mutanten gram-negativer Bakterien durchführt, die ein Re-Lipopolysaccharid der Formel I

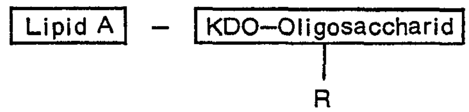

(I)

synthetisieren, in der das KDO-Oligosaccharid zwei oder drei KDO-Gruppen enthält und mindestens eine dieser KDO-Gruppen mit dem Rest R substituiert ist, der Lipid-A-Bestandteil ein Glukosamin-Disaccharid enthält, das an den Hydroxylgruppen verschiedenartig substituiert ist durch Fettsäurereste, 4-Aminoarabinosephosphatreste und durch Diphosphoryläthanolaminreste, und das an den Aminogruppen durch Fettsäurereste substituiert ist, KDO eine 3-Desoxy-D-mannoseoctulosonsäure der Formel II

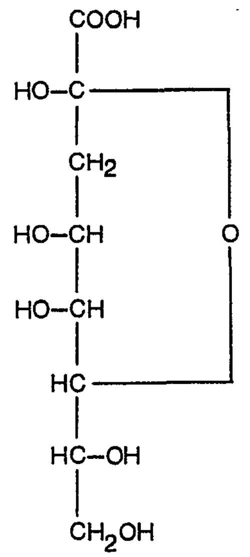

(II)

bedeutet, und in der R ein Wasserstoffatom, eine Phosphatgruppe oder eine Diphosphorylniederalkanolamingruppe ist, und das Re-LPS der Formel I mit Chlamydia-Glykolipiden kreuzreagiert.

3. Verfahren nach Anspruch 1 oder 2, dadurch gekennzeichnet, daß man die Verbindung der Formel I mit Alkali behandelt.

4. Verfahren zur Herstellung gruppenspezifischer Chlamydia-Antikörper, dadurch gekennzeichnet, daß man Re-Mutanten gram-negativer Bakterien, deren LPS nicht isoliert worden war, bis zur logarithmischen Phase züchtet, durch Zentrifugieren sammelt, mit physiologischer Kochsalzlösung wäscht, in physiologischer Kochsalzlösung suspendiert, Hitze- und Formalin-inaktiviert, in Kaninchen injiziert und die Antikörper aus dem Kaninchenserum gewinnt.

5. Verfahren nach Anspruch 4, dadurch gekennzeichnet, daß man isoliertes LPS in einem Komplex mit einem Trägerprotein zur Immunisierung verwendet.

6. Verwendung des Präparats nach einem der Ansprüche 1 bis 3 zum Nachweis gruppenspezifischer Chlamydia-Antikörper durch serologische oder immunologische Verfahren.

**Revendications pour les Etats contractants: BE CH DE FR GB IT LI NL SE**

1. Préparation pour le diagnostic d'infections chlamydiales et pour la production d'anticorps spécifiques du groupe des chlamydiacées, contenant un lipopolysaccharide de chimiotype Re isolé de mutants Re de bactéries gram négatives, réagissant de façon croisée (réagissant dans les deux directions) avec un glycolipide chlamydial.

2. Préparation suivant la revendication 1, caractérisée en ce qu'elle a été isolée à partir de mutants Re de bactéries gram négatives qui synthétisent un Re-lipopolysaccharide tel qu'illustré par la formule I:

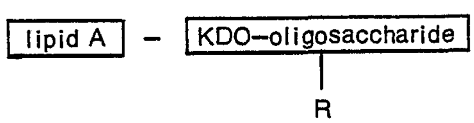

(I)

dans laquelle le KDO-oligosaccharide contient deux ou trois groupes KDO, au moins l'un de ces groupes KDO étant substitué par R, le composant lipide A contient un disaccharide du glucosamine qui est substitué de diverses manières à l'endroit des groupes hydroxyle par des groupes d'acide gras, des groupes 4-aminoarabinose phosphate et des groupes diphosphoryléthanolamine, et qui est substitué à l'endroit des groupes amino par des groupes d'acide gras, KDO désignant l'acide 3-désoxy-D-mannose-octulosonique de la formule II:

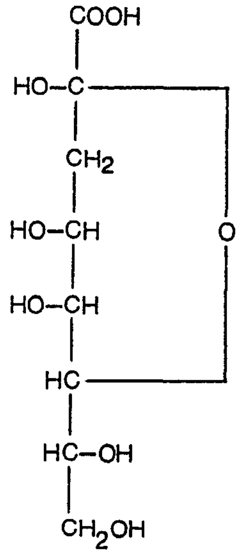

(II)

et R désignant un atome d'hydrogène, un groupe phosphate ou un groupe diphosphoryl alcanolamine inférieur, le Re-LPS représenté par la formule I réagissant de façon croisée avec les glycolipides chlamydiaux.

3. Préparation suivant l'une ou l'autre des revendications 1 et 2, caractérisée en ce que le produit représenté par la formule I a été traité avec un alcali.

4. Préparation pour la production d'anticorps spécifiques du groupe des chlamydiacées, caractérisée en ce qu'elle comprend des mutants Re de bactéries gram négatives dont le LPS n'a pas été isolé.

5. Utilisation de la préparation suivant l'une quelconque des revendications 1 à 3 pour la production d'anticorps spécifiques du groupe des chlamydiacées.

6. Utilisation de la préparation suivant l'une quelconque des revendications 1 à 3 pour la détection d'anticorps spécifiques du groupe des chlamydiacées par des méthodes sérologiques ou immunologiques.

**Revendications pour l'Etat contractant: AT**

1. Procédé de production d'une préparation pour le diagnostic d'infections chlamydiales et pour la production d'anticorps spécifiques du groupe des chlamydiacées, caractérisé en ce que l'on isole un lipopolysaccharide de chimiotype Re réagissant de façon croisée (réagissant dans les deux directions) avec un glycolipide chlamydial de mutants Re de bactéries gram négatives.

2. Procédé suivant la revendication 1, caractérisé en ce que l'on effectue l'isolement à partir de mutants R de bactéries gram négatives qui synthétisent un Re-lipopolysaccharide tel qu'illustré par la formule I:

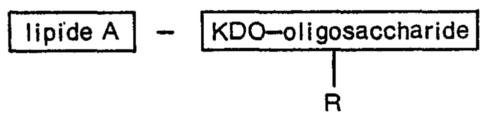

(I)

dans laquelle le KDO-oligosaccharide contient deux ou trois groupes KDO, au moins l'un de ces groupes KDO étant substitué par R, le composant lipide A contenant un disaccharide de glucosamine qui est substitué de diverses manières à l'endroit des groupes hydroxyle par des groupes d'acide gras, des groupes 4-aminoarabinose phosphate et des groupes diphosphoryléthanolamine, et qui est substitué à l'endroit des groupes amino par des groupes d'acide gras, KDO signifiant l'acide 3-désoxy-D-mannose-octulosonique de la formule II:

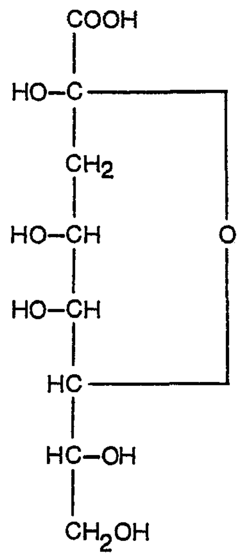

(II)

et R désignant un atome d'hydrogène, un groupe phosphate ou un groupe diphosphoryl alcanolamine inférieur, le Re-LPS représenté par la formule I réagissant de façon croisée avec les glycolipides chlamydiaux.

3. Procédé suivant l'une ou l'autre des revendications 1 et 2, caractérisé en ce que l'on traite le produit de la formule I par un alcali.

4. Procédé de production d'anticorps spécifiques du groupe des chlamydiacées, caractérisé en ce que l'on fait croître les mutants Re de bactéries gram négatives, dont le LPS n'a pas été isolé, jusqu'à la phase logarithmique, on les recueille par centrifugation, on les lave au moyen d'une solution saline physiologique, on les met en suspension dans une solution saline physiologique, on les inactive par de la chaleur et de la formaline et on les injecte à des lapins, les anticorps étant recueillis du sérum des lapins.

5. Procédé suivant la revendication 4, caractérisé en ce que l'on utilise du LPS isolé complexé par une protéine de support pour l'immunisation.

6. Utilisation de la préparation suivant l'une quelconque des revendications 1 à 3 pour la détection d'anticorps spécifiques du groupes des chlamydiacées par des méthodes sérologiques ou immunologiques.

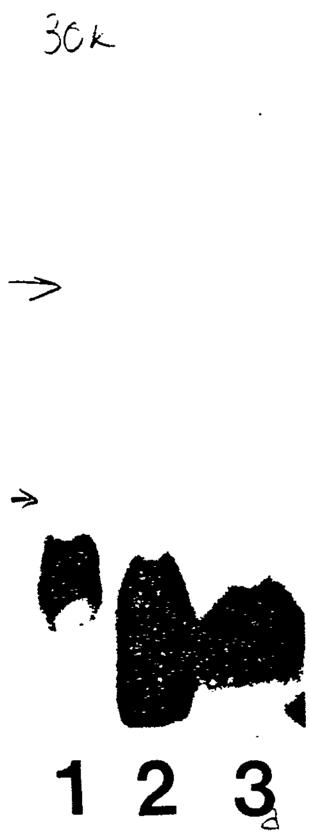


Fig. 1

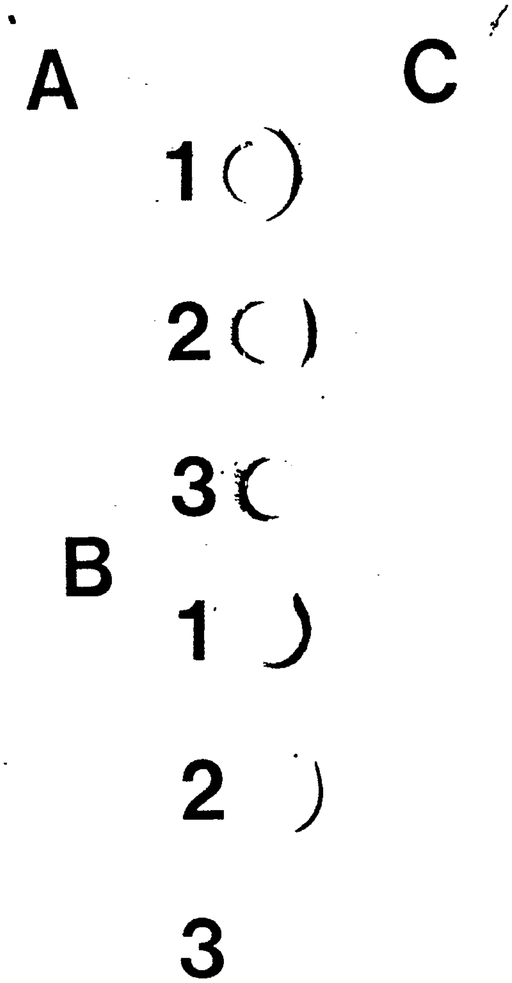


Fig. 2

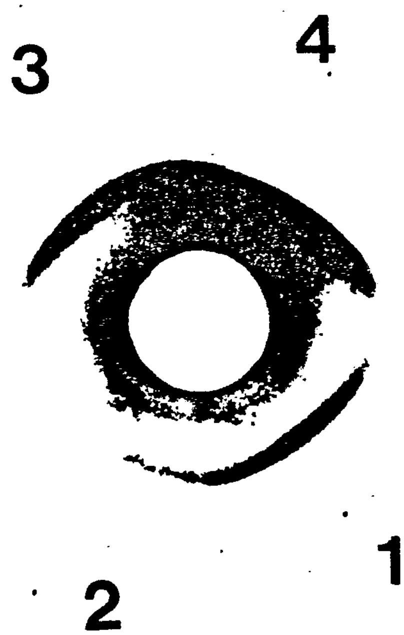


Fig. 3